# EUROPÄISCHE PATENTSCHRIFT

(11) **EP 0 884 299 B1**
(45) Veröffentlichungstag und Bekanntmachung des Hinweises auf die Patenterteilung: **23.10.2002**
(21) Anmeldenummer: 98109916.1
(22) Anmeldetag: 29.05.1998
(51) Int. Cl.: C07C 51/377, C07C 53/126

(54) **Verfahren zur Herstellung von 3,3-Dimethylbuttersäure**
Process for the synthesis of 3,3-dimethyl-butyric acid
Procédé de fabrication de l'acide 3,3-diméthyl butyrique

(30) Priorität: 11.06.1997 DE 19724584
(43) Veröffentlichungstag der Anmeldung: 16.12.1998
(73) Patentinhaber: BAYER AG, 51368 Leverkusen (DE)
(72) Erfinder: Stelzer, Uwe, Dr., 51399 Burscheid (DE)

(56) Entgegenhaltungen:
- US-A- 2 004 066
- STILLER E.T. ET AL.: "SYNTHESIS AND ANTIINFLAMMATORY ACTIVITIES OF ALPHA-METHYLFLUORENE-2-ACETIC ACID AND RELATED COMPOUNDS" JOURNAL OF MEDICINAL CHEMISTRY, Bd. 15, Nr. 10, 1972, Seiten 1029-1032, XP002077469
- HUANG-MINLON: "A SIMPLE MODIFICATION OF THE WOLFF-KISHNER REDUCTION" JOURNAL OF THE AMERICAN CHEMICAL SOCIETY, Bd. 68, 1946, Seiten 2487-2488, XP002077470

## Beschreibung

Die vorliegende Erfindung betrifft ein neues Verfahren zur Herstellung von 3,3-Dimethylbuttersäure.

Es ist bekannt, daß man durch Umsetzung von tert.-Butanol bzw. tert.-Butylchlorid mit Vinylidenchlorid in Schwefelsäure und BF₃ die 3,3-Dimethylbuttersäure erhält. Problematisch hierbei ist die technische Verfügbarkeit von 1,1-Dichlorethen und der Umgang mit BF₃. Ferner ist bekannt, daß 2-Aryl-2-oxocarbonsäuren durch Umsetzung mit Hydrazinhydrat und anschließendem Erhitzen mit Basen (Wolff-Kishner-Reduktion) zu 2-Arylessigsäuren reduziert werden können (siehe z.B. Monath. Chem. 1952, 83, 883, J. Med. Chem. 1972, 15, 1029), J. Heterocycl. Chem. 1990, 27, 1489 und JACS 1946, 68, 2487-2488).

Aufgabe der vorliegenden Erfindung war es, ein einfaches, technisch realisierbares Verfahren zur Herstellung von 3,3-Dimethylbuttersäure bereitzustellen.

Gegenstand der vorliegenden Erfindung ist ein Verfahren zur Herstellung von 3,3-Dimethylbuttersäure der Formel (I) 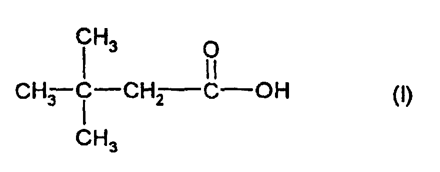 das dadurch gekennzeichnet ist, daß man Trimethylbrenztraubensäure der Formel (II) 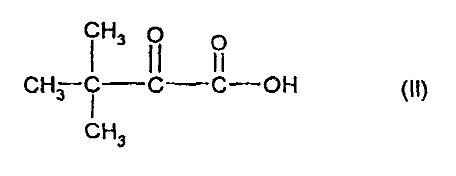 gegebenenfalls in Gegenwart eines Verdünnungsmittels bei Temperaturen zwischen 0°C und 200°C mit Hydrazinhydrat zum Hydrazon der Formel (III) 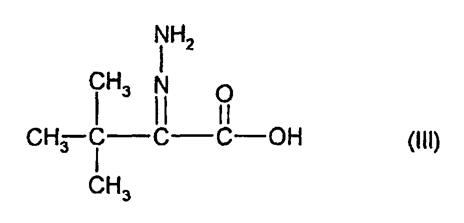 umsetzt und dieses anschließend gegebenenfalls in Gegenwart eines Verdünnungsmittels bei Temperaturen zwischen 100°C und 280°C mit einer Base behandelt.

Überraschenderweise liefert das erfindungsgemäße Verfahren 3,3-Dimethylbuttersäure selektiv und in hohen Ausbeuten, da nach dem Stand der Technik zu erwarten war, daß die tert.-Butylglyoxylsäure beim Erhitzen mit Aminderivaten (z.B. Anilin) sehr leicht zum Trimethylacetaldehyd decarboxyliert (Houben-Weyl, Methoden der organischen Chemie, Band VII/1, Seite 320).

Weiterhin führt die thermische Abspaltung von CO₂ bei 200 bis 220°C zu einem hohen Anteil an Pivalsäure (J. Org. Chem. 35, 3726 (1970)). Dies deckt sich auch mit der generellen Aussage, daß α-Ketocarbonsäuren sehr leicht decarboxylieren (Houben-Weyl, Band VII/1, Seite 317).

Verwendet man z.B. Triglykol als Verdünnungsmittel und Kaliumhydroxid als Base, so kann der Verlauf des erfindungsgemäßen Verfahrens durch das folgende Formelschema wiedergegeben werden:

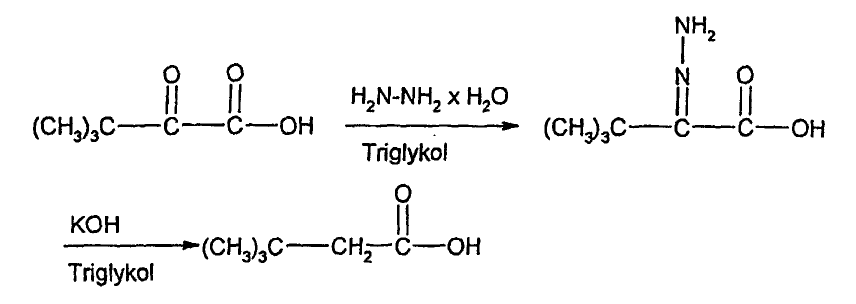

Die erfindungsgemäß hergestellte 3,3-Dimethylbuttersäure der Formel (I) ist bekannt (siehe z.B. J. Org. Chem. 6, 132 (1941) und US 2.004.066).

Die als Ausgangsstoff verwendete Trimethylbrenztraubensäure der Formel (II) ist bekannt (siehe Houben Weyl, Methoden der Organischen Chemie, Band VII/1, Seite 320) und käuflich bzw. kann in einfacher Weise hergestellt werden, indem man Pinakolin zur tert.-Butylglyoxylsäure oxidiert.

Ebenfalls bekannt ist das Hydrazon der Formel (III) (siehe J. Prakt. Chem. 152, 324 (1939)) und Hydrazinhydrat.

Als Verdünnungsmittel für die erste Stufe des erfindungsgemäßen Verfahrens kommen die folgenden Lösungsmittel in Frage:

Alkohole, insbesondere Polyalkohole wie Diglykol und Triglykol (Triethylenglykol), ferner Methanol, Ethanol, o- oder i-Propanol, n-, i-, sek- oder tert.-Butanol, Octanol, Hexanol etc., Ethandiol, Propan-1,2-diol, Ethoxyethanol, Methoxyethanol, Diethylenglykolmonomethylether, Diethylenglykolmonoethylether oder Sulfoxide, wie Dimethylsulfoxid; Sulfone, wie Sulfolan oder deren Gemische mit Wasser.

Bevorzugt werden Triglykol und Diglykol verwendet.

Als Verdünnungsmittel für die zweite Stufe des erfindungsgemäßen Verfahrens kommen folgende Lösungsmittel in Frage:
Alkohole, insbesondere Polyalkohole wie Diglykol und Triglykol (Triethylenglykol), ferner Methanol, Ethanol, o- oder i-Propanol, n-, i-, sek- oder tert.-Butanol, Octanol, Hexanol etc., Ethandiol, Propan-1,2-diol, Ethoxyethanol, Methoxyethanol, Diethylenglykolmonomethylether, Diethylenglykolmonoethylether oder Sulfoxide, wie Dimethylsulfoxid.

Bevorzugt werden Diglykol und Triethylenglykol verwendet.

Als Base für die Durchführung der zweiten Stufe des erfindungsgemäßen Verfahrens werden vorzugsweise Alkalihydroxide oder Alkalialkoholate verwendet. Genannt seien beispielsweise Natriumhydroxid, Kaliumhydroxid, Natriummethanolat und Natriumethanolat und K-tert.-Butanolat.

Bevorzugt werden Kaliumhydroxid oder Natriumhydroxid verwendet.

Die erste Stufe des erfindungsgemäßen Verfahrens wird bei Temperaturen zwischen 0°C und 200°C, bevorzugt zwischen 20°C und 180°C durchgeführt.

Die zweite Stufe des erfindungsgemäßen Verfahrens wird bei Temperaturen zwischen 100°C und 280°C, bevorzugt zwischen 100°C und 250°C durchgeführt.

Beide Stufen werden in der Regel unter Normaldruck durchgeführt.

Bei der Durchführung des erfindungsgemäßen Verfahrens setzt man in der ersten Stufe auf 1 mol der Verbindung der Formel (II) 1 bis 10 mol, bevorzugt 1 bis 7 mol Hydrazinhydrat ein.

In der zweiten Stufe setzt man auf 1 mol der Verbindung der Formel (III) 1 bis 10 mol, bevorzugt 1 bis 7 mol Base ein.

Die erste Stufe des erfindungsgemäßen Verfahrens wird im allgemeinen so durchgeführt, daß Trimethylbrenztraubensäure zusammen mit Hydrazinhydrat in einem geeigneten Verdünnungsmittel erhitzt wird, bis die Reaktion beendet ist, wobei gegebenenfalls Wasser durch azeotropieren entfernt wird.

Zur Aufarbeitung wird die alkoholische Lösung im Vakuum zur Trockene eingedampft. Das angefallene Hydrazon kann in der Regel direkt in die zweite Stufe eingesetzt werden.

Die zweite Stufe des erfindungsgemäßen Verfahrens wird im allgemeinen so durchgeführt, daß die gemäß der ersten Stufe erhaltene Verbindung der Formel (III) zusammen mit der angegebenen Menge Base in einem geeigneten Verdünnungsmittel auf die angegebene Temperatur erhitzt wird, bis die Gasentwicklung (Stickstoffabspaltung) beendet ist.

Zur Aufarbeitung wird beispielsweise mit Wasser versetzt, angesäuert, das Produkt mit einem geeigneten Extraktionsmittel extrahiert und anschließend destilliert.

Als Extraktionsmittel kommen beispielsweise in Frage:
aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-butylether, Methyl-t-Amylether, 1,2-Dimethoxyethan oder 1,2-Diethoxyethan; Nitrile, wie Acetonitril, Propionitril, n- oder i-Butyronitril oder Benzonitril; Ester wie Essigsäuremethylester oder Essigsäureethylester.

Nach einer besonderen Ausführungsform des erfindungsgemäßen Verfahrens wird das Zwischenprodukt der Formel (III) nicht isoliert, sondern die erste und die zweite Stufe werden zusammengefaßt und als Eintopfreaktion durchgeführt.

Dabei geht man beispielsweise so vor, daß man die Verbindung der Formel (II), Hydrazinhydrat und die Base in einem geeigneten Verdünnungsmittel (siehe oben) unter Rückfluß erhitzt, bis sich kein Hydrazon mehr bildet, überschüssiges Hydrazinhydrat abdestilliert und den Rückstand weiter erhitzt, bis die Gasentwicklung beendet ist. Dann wird beispielsweise wie oben beschrieben aufgearbeitet.

3,3-Dimethylbuttersäure kann als Zwischenprodukt für die Synthese insektizider, fungizider bzw. herbizider Wirkstoffe verwendet werden (siehe z.B. EP-A-0 528 156).

### Beispiel

28 g Trimethylbrenztraubensäure, 30 g Hydrazinhydrat und 62,8 g Kaliumhydroxid in 200 ml Triglykol (Triethylenglykol) werden 2 Stunden auf Rückflußtemperatur erhitzt. Anschließend wird überschüssiges Hydrazinhydrat abdestilliert und bis zum Ende der Gasentwicklung erhitzt. Die Innentemperatur erreicht 190 bis 200°C. Nach dem Abkühlen wird mit 300 ml H₂O versetzt und mit konzentrierter Salzsäure auf pH 2 gestellt. Die wäßrige Phase wird mehrmals mit Toluol extrahiert und die vereinigten organischen Phasen fraktioniert destilliert. Man isoliert 22,4 g (90% der Theorie) 3,3-Dimethylbuttersäure bei 66 bis 68°C und 8 mbar.

## Patentansprüche

1. Verfahren zur Herstellung von 3,3-Dimethylbuttersäure der Formel (I) 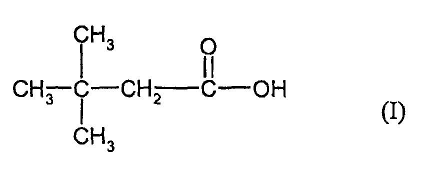 **dadurch gekennzeichnet, daß** man Trimethylbrenztraubensäure der Formel (II) 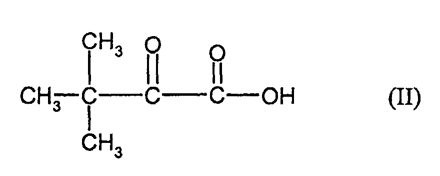 gegebenenfalls in Gegenwart eines Verdünnungsmittels bei Temperaturen zwischen 0°C und 200°C mit Hydrazinhydrat zum Hydrazon der Formel (III) 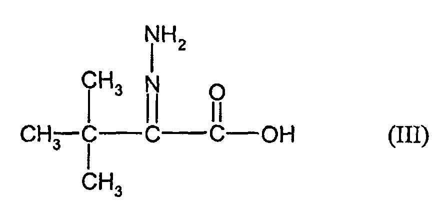 umsetzt und dieses anschließend gegebenenfalls in Gegenwart eines Verdünnungsmittels bei Temperaturen zwischen 100°C und 280°C mit einer Base behandelt.

2. Verfahren zur Herstellung von 3,3-Dimethylbuttersäure der Formel (I) gemäß Anspruch 1, **dadurch gekennzeichnet, dass** man Trimethylbrenztraubensäure gegebenenfalls in Gegenwart eines Verdünnungsmittels mit Hydrazinhydrat und einer Base umsetzt.

## Claims

1. Process for preparing 3,3-dimethylbutyric acid of the formula (I), 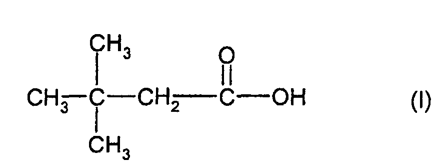 **characterized in that** trimethylpyruvic acid of the formula (II) 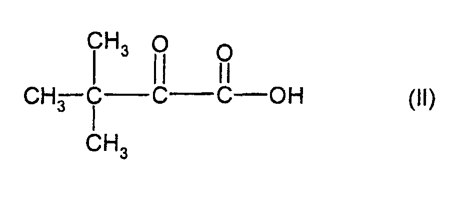 is, if appropriate in the presence of a diluent, reacted with hydrazine hydrate at temperatures between 0°C and 200°C to give the hydrazone of the formula (III) 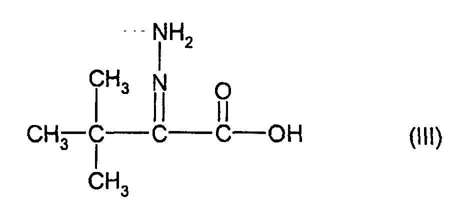 and this is subsequently, if appropriate in the presence of a diluent, treated with a base at temperatures between 100°C and 280°C.

2. Process for preparing 3,3-dimethylbutyric acid of the formula (I) according to Claim 1, **characterized in that** trimethylpyruvic acid is, if appropriate in the presence of a diluent, reacted with hydrazine hydrate and a base.

## Revendications

1. Procédé de préparation d' acide 3,3-diméthylbutyrique de formule (I) 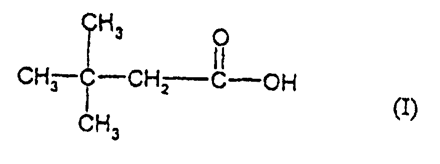 **caractérisé en ce qu'**on fait réagir de l'acide triméthylpyruvique de formule (II) 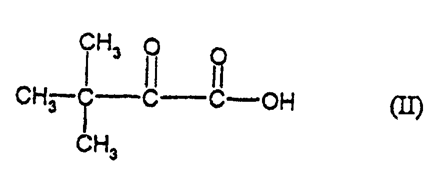 le cas échéant en présence d'un diluant à des températures entre 0°C et 200°C avec de l'hydrate d'hydrazine en hydrazone de formule (III) 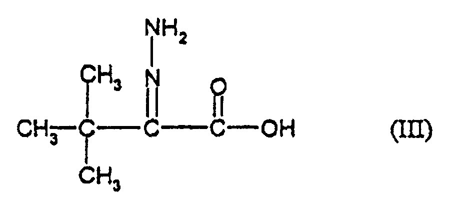 et qu'on traite ensuite celle-ci le cas échéant en présence d'un diluant à des températures entre 100°C et 280°C avec une base.

2. Procédé de préparation d'acide 3,3-diméthylbutyrique de formule (I) selon la revendication 1, **caractérisé en ce qu'**on fait réagir de l'acide triméthylpyruvique le cas échéant en présence d'un diluant avec de l'hydrate d'hydrazine et une base.
